(19) Europäisches Patentamt
European Patent Office
Office européen des brevets

(11) EP 4 211 175 B1

(12) # FASCICULE DE BREVET EUROPEEN

(45) Date de publication et mention de la délivrance du brevet:
**23.10.2024 Bulletin 2024/43**

(21) Numéro de dépôt: **21769773.9**

(22) Date de dépôt: **19.08.2021**

(51) Classification Internationale des Brevets (IPC):
***C08F 2/10*** (2006.01) ***C08F 20/58*** (2006.01)
***C08F 220/56*** (2006.01) ***C02F 1/56*** (2023.01)
***C02F 1/28*** (2023.01) ***C11D 3/37*** (2006.01)
***A61K 8/81*** (2006.01) ***A61Q 19/00*** (2006.01)
***C09K 8/588*** (2006.01) ***D21H 17/37*** (2006.01)

(52) Classification Coopérative des Brevets (CPC):
(C-Sets disponibles)
**C08F 20/58; A61K 8/8158; A61Q 19/00; C02F 1/285; C02F 1/56; C08F 2/10; C08F 220/56; C09K 8/588; C11D 3/378; D21H 17/37;**
A61K 2800/48; A61K 2800/49; C09K 2208/28

(Cont.)

(86) Numéro de dépôt international:
**PCT/FR2021/051475**

(87) Numéro de publication internationale:
**WO 2022/053752 (17.03.2022 Gazette 2022/11)**

(54) **POLYMERE D'ACIDE 2-ACRYLAMIDO-2-METHYLPROPANE SULFONIQUE OU DE SES SELS**

POLYMER DER 2-ACRYLAMIDO-2-METHYLPROPANSULFONSÄURE ODER IHRER SALZE

POLYMER OF 2-ACRYLAMIDO-2-METHYLPROPANE SULFONIC ACID OR ITS SALTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.09.2020 FR 2009216**

(43) Date de publication de la demande:
**19.07.2023 Bulletin 2023/29**

(73) Titulaire: **SNF Group**
**42160 Andrézieux-Bouthéon (FR)**

(72) Inventeurs:
- **FAVERO, Cédrick**
**42160 ANDREZIEUX-BOUTHEON (FR)**
- **KIEFFER, Johann**
**42160 ANDREZIEUX-BOUTHEON (FR)**
- **LEGRAS, Benoît**
**42160 ANDREZIEUX-BOUTHEON (FR)**

(74) Mandataire: **Cabinet Laurent & Charras**
**Le Contemporain**
**50 Chemin de la Bruyère**
**69574 Dardilly Cedex (FR)**

(56) Documents cités:
**EP-A1- 3 350 159**
**EP-B1- 3 350 159**
**WO-A1-2018/172676**
**JP-A- 2010 270 168**
**JP-A- 2010 270 169**
**JP-A- 2010 270 170**
**JP-A- 2011 046 612**
**JP-A- 2015 504 441**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC):
(Cont.)

C-Sets
**C08F 220/56, C08F 220/585;**
**C08F 220/56, C08F 220/585, C08F 220/585**

## Description

### Domaine de l'invention

La présente invention concerne un polymère d'acide 2-acrylamido-2-méthylpropane sulfonique ou d'au moins un de ses sels. Plus précisément, l'invention concerne un polymère obtenu à partir du monomère acide 2-acrylamido-2-méthylpropane sulfonique ou d'au moins un de ses sels, ledit monomère contenant des impuretés.

### Etat antérieur de la technique

L'acide 2-acrylamido-2-méthylpropane sulfonique (ATBS) et ses sels sont largement utilisés comme matière première pour obtenir des polymères utilisés en tant que dispersant, épaississant, floculant ou superabsorbant dans divers secteurs comme l'industrie pétrolière, la construction, le textile, le traitement des eaux (dessalement de l'eau de mer, industrie minérale, etc...) ou la cosmétique.

La réaction mise en oeuvre dans le procédé de synthèse de l'acide 2-acrylamido-2-méthylpropane sulfonique répond au schéma réactionnel ci-dessous, dans lequel l'acrylonitrile est présent en excès de manière à être à la fois le solvant de la réaction et un réactif. L'acrylonitrile est mis en contact avec de l'acide sulfurique fumant (oléum) et de l'isobutylène.

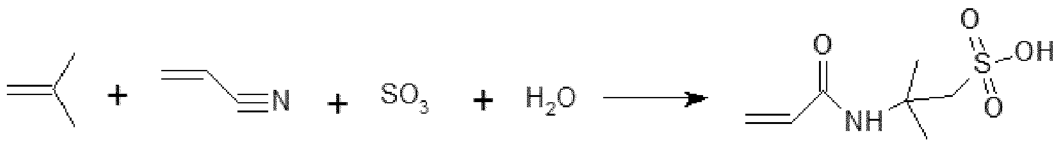

L'acide 2-acrylamido-2-méthylpropane sulfonique (ATBS) n'est pas soluble dans le solvant acrylonitrile. Par conséquent, le produit de réaction est sous une forme de suspension de cristaux dans le solvant de réaction.

L'acide 2-acrylamido-2-méthylpropane sulfonique est par la suite séparé de l'acrylonitrile, généralement par filtration, puis séché. Le séchage de l'acide acrylamido-2-méthyl-2-propane sulfonique est nécessaire afin de diminuer la quantité d'acrylonitrile et d'acrylamide restant présent dans le cristal.

Par la suite, l'acide 2-acrylamido-2-méthylpropane sulfonique est purifié afin d'éliminer les impuretés contenues dans le monomère. Il existe de nombreuses techniques de purification qui permettent de diminuer la présence de ces impuretés. Cette purification permet ainsi d'éviter la contamination du monomère qui, selon les connaissances actuelles, nuit à la bonne polymérisation du monomère.

Il existe de nombreuses méthodes de purification de l'acide 2-acrylamido-2-méthylpropane sulfonique. Le plus souvent l'acide 2-acrylamido-2-méthylpropane sulfonique est redissous dans un solvant à chaud, afin d'obtenir une solution saturée. Lors du refroidissement des cristaux de haute pureté sont obtenus. Au fil du temps, différents solvants ont été utilisés pour améliorer cette purification. On peut citer l'acide acétique, les alcools en C1-C4, les cétones ou l'acide propionique.

Le document US 2010/0274048 identifie deux types d'impuretés présentes dans l'acide 2-acrylamido-2-méthylpropane sulfonique, l'acide 2-méthyl-2-propényl-sulfonique (IBSA) et l'acide 2-méthylidène-1,3- propylènedisulfonique (IBDSA). Selon les auteurs, ces deux impuretés agissent comme des agents de transfert de chaine et affectent fortement la polymérisation lorsqu'elles sont présentes au-delà d'une certaine concentration dans le monomère acide 2-acrylamido-2-méthylpropane sulfonique.

Pour résoudre ce problème, le document décrit un procède de fabrication d'acide 2-acrylamido-2-méthylpropane sulfonique dans lequel les conditions de réaction sont optimisées de manière que la concentration en trioxyde de soufre est réduite, ce qui a pour effet de réduire le taux de ces impuretés en dessous de 100 ppm, préférentiellement en dessous de 30 ppm.

Selon les auteurs, l'acide 2-acrylamido-2-méthylpropane sulfonique non purifié, et par conséquent contenant des quantités d'impuretés élevées, ne peut pas être utilisé tel quel pour obtenir des polymères d'acide 2-acrylamido-2-méthylpropane sulfonique ayant un poids moléculaire élevé.

Les documents JP 2011-046612 et JP 2015-504441 décrivent deux méthodes de production d'ATBS présentant des quantités d'IBSA et éventuellement d'IBDSA inférieures ou égales à 100 ppm.

Les documents JP 2010-270169, JP 2010-270170 et JP 2010-270168 décrivent des polymères obtenus à partir d'ATBS contenant jusqu'à 120 ppm d'IBSA. Les exemples comparatifs de ces documents montrent que la présence de 200 ppm d'IBSA est néfaste.

Il aurait donc été attendu que la polymérisation d'acide 2-acrylamido-2-méthylpropane sulfonique non purifié ou contenant un taux d'impuretés élevé conduise à des polymères ayant des propriétés dégradées. Cependant, contre

toute attente, la Demanderesse a découvert qu'il était possible d'utiliser de l'acide 2-acrylamido-2-méthylpropane sulfonique contenant une quantité substantielle d'impuretés et d'obtenir des polymères de haut poids moléculaire sans que leurs propriétés ne soient significativement affectées.

## Exposé de l'invention

La présente invention concerne un polymère obtenu au moins à partir d'une quantité A d'acide 2-acrylamido-2-méthylpropane sulfonique, sous forme acide et/ou salifiée. La quantité A d'acide 2-acrylamido-2-méthylpropane sulfonique contient de 250 à 20 000 ppm en poids d'acide 2-méthyl-2-propényl-sulfonique, sous forme acide et/ou salifiée, avantageusement 300 à 20 000 ppm et entre 300 et 10 000 pp, em poids d'acide 2-méthylidène-1,3-propylènedisulfonique.

L'invention a également pour objet l'utilisation dudit polymère dans un domaine choisi parmi la récupération du pétrole et du gaz, le traitement de l'eau, le traitement des boues, la fabrication du papier, la construction, l'industrie minière, la formulation de produits cosmétiques, la formulation de détergents, la fabrication du textile, et l'agriculture. L'invention a aussi pour objet l'utilisation dudit polymère comme floculant, agent réducteur de viscosité, agent épaississant, agent absorbant ou agent réducteur de friction. Le polymère selon l'invention est préférentiellement un polymère hydrosoluble ou un polymère superabsorbant.

Sauf indication contraire, les ppm et les pourcentages sont exprimés en poids.

Par définition un polymère hydrosoluble est un polymère qui donne une solution aqueuse lorsqu'il est dissous sous agitation et avec une concentration de 10 g/L dans l'eau à 25°C. La quantité d'impureté d'acide 2-méthyl-2-propényl-sulfonique et/ou de ses sels (IBSA) est exprimée en ppm en poids dans le monomère d'acide 2-acrylamido-2-méthylpropane sulfonique et/ou de ses sels. De manière générale, sauf indication contraire, par « acide 2-acrylamido-2-méthylpropane sulfonique » ou ATBS on désigne la forme acide et/ou la forme salifiée. Il en est de même avec l'acide 2-méthyl-2-propényl-sulfonique (IBSA) et pour l'acide 2-méthylidène-1,3- propylènedisulfonique (IBDSA). La forme acide d'un acide correspond à la forme de l'acide de Brønsted alors que la forme salifiée (ou un sel) correspond à la base de Brønsted de l'acide, le contre-ion étant avantageusement choisi parmi les métaux alcalins, les métaux alcalino-terreux et les ammoniums (primaire, secondaire, tertiaire ou quaternaire).

Selon l'invention, il est possible d'obtenir des polymères de haut poids moléculaire à partir de monomère d'acide 2-acrylamido-2-méthylpropane sulfonique contenant une quantité importante d'impureté IBSA sans que les performances des polymères ne soient significativement et négativement impactées.

Selon un mode préféré, le polymère est obtenu à partir d'une quantité A d'acide 2-acrylamido-2-méthylpropane sulfonique, sous forme acide et/ou salifiée, contenant de 300 à 10 000 ppm en poids d'acide 2-méthyl-2-propényl-sulfonique, sous forme acide et/ou salifiée, plus particulièrement 350 à 5000 ppm, encore plus particulièrement 400 à 2000 ppm, et encore plus particulièrement 500 à 1500 ppm.

L'acide 2-acrylamido-2-méthylpropane sulfonique (forme acide et/ou salifiée), utilisé pour préparer le polymère, peut être le produit d'un procédé de fabrication d'acide 2-acrylamido-2-méthylpropane sulfonique à l'issue duquel il n'est substantiellement pas purifié. De manière préférentielle il n'est pas purifié. Il peut aussi être un résidu ou un déchet ou une purge d'un procédé de purification d'acide 2-acrylamido-2-méthylpropane sulfonique. De manière générale, le procédé de purification est avantageusement effectué par un procédé de recristallisation dans un solvant comme l'acrylonitrile, l'acide acétique ou le méthanol. Il peut aussi être également la purge ou le filtrat obtenu lors du procédé de production de l'ATBS, par exemple selon le procédé décrit dans le document WO 2018/172676. Le résidu ou déchet ou purge ou filtrat contient une quantité variable d'ATBS. Il peut s'agir de la quantité A d'ATBS, ou de la quantité B d'ATBS (voir ci-après), ou de la somme des quantités A et B, d'ATBS ou de la quantité totale d'ATBS utilisé pour préparer le polymère.

Ainsi, selon l'invention il est possible de polymériser un monomère d'acide 2-acrylamido-2-méthylpropane sulfonique considéré comme étant de basse qualité puisque contenant un taux d'impuretés élevés et d'obtenir un polymère de haut poids moléculaire sans que les caractéristiques du polymère ne soient substantiellement affectées. Autrement dit, là où il semblait nécessaire de purifier l'acide 2-acrylamido-2-méthylpropane sulfonique, la présente invention démontre qu'il est possible de réduire le niveau d'exigence lors d'une étape de purification, idéalement, de se passer d'étape de purification de l'acide 2-acrylamido-2-méthylpropane sulfonique, et d'obtenir des polymères de haut poids moléculaire présentant des performances satisfaisantes.

La quantité A d'acide 2-acrylamido-2-méthylpropane sulfonique, sous forme acide et/ou salifiée, contient entre 300 et 10 000 ppm en poids d'acide 2-méthylidène-1,3- propylènedisulfonique (IBDSA), sous forme acide et/ou salifiée, plus particulièrement entre 350 et 5000 ppm, encore plus particulièrement entre 400 et 2000 ppm, encore plus particulièrement entre 500 et 1500 ppm.

De manière tout à fait surprenante, la Demanderesse a remarqué qu'il était ainsi possible d'obtenir des polymères de haut poids moléculaire, même en présence d'une quantité substantielle d'IBSA et éventuellement d'IBDSA.

Lorsque le polymère est un polymère hydrosoluble, il a un poids moléculaire moyen en poids avantageusement

supérieur à 100 000 g/mol, plus avantageusement supérieur à 500 000 g/mol, préférentiellement supérieur à 1 million de g/mol, plus préférentiellement supérieur à 1,5 million, encore plus préférentiellement supérieur à 2 millions, encore plus préférentiellement supérieur à 3 millions, encore plus préférentiellement compris entre 5 et 40 millions, encore plus préférentiellement compris entre 7 et 30 millions, encore plus préférentiellement compris entre 9 et 30 millions, encore plus préférentiellement compris entre 10 et 25 millions, encore plus préférentiellement supérieur à 12 millions.

Les polymères superabsorbants ont par définition un poids moléculaire infini puisqu'ils forment un réseau tridimensionnel. Un superabsorbant est un polymère réticulé capable, lorsqu'il est sous forme de particule solide ou gélifiée, d'absorber une quantité importante d'eau, généralement d'au moins une fois son poids. Ils sont obtenus avec les mêmes quantités d'ATBS que les polymères hydrosolubles.

Le poids moléculaire moyen en poids du polymère selon l'invention est avantageusement déterminé par la viscosité intrinsèque du polymère. La viscosité intrinsèque peut être mesurée par des méthodes connues de la personne du métier et peut être calculée à partir des valeurs de viscosité réduite pour différentes concentrations en polymère par méthode graphique consistant à relever les valeurs de viscosité réduite (axe des ordonnées) sur la concentration (axe des abscisses) et d'extrapoler la courbe jusqu'à concentration nulle. La valeur de viscosité intrinsèque est relevée sur l'axe des ordonnées ou en utilisant la méthode des moindres carrés. Le poids moléculaire peut alors être déterminé par l'équation de Mark-Houwink :

$$[\eta] = K\ M^{\alpha}$$

dans laquelle

$[\eta]$ représente la viscosité intrinsèque du polymère déterminée par la méthode de mesure de viscosité en solution,
K représente une constante empirique,
M représente le poids moléculaire du polymère,
$\alpha$ représente le coefficient de Mark-Houwink,
K et $\alpha$ dépendent du système particulier polymère-solvant.

Le polymère selon l'invention peut être un homopolymère d'acide 2-acrylamido-2-méthylpropane sulfonique ou un copolymère comprenant en outre au moins un monomère choisi dans le groupe comprenant les monomères non ioniques, les monomères anioniques, les monomères cationiques, les monomères zwittérioniques, et leurs mélanges.

Le polymère selon l'invention est préférentiellement obtenu à partir d'une quantité totale d'acide 2-acrylamido-2-méthylpropane sulfonique (forme acide et/ou salifiée) représentant, par rapport à la quantité totale de monomères, entre 1 et 100% molaire, préférentiellement plus de 10% molaire, plus préférentiellement plus de 20% molaire, encore plus préférentiellement plus de 30% molaire Cette quantité peut être supérieure à 40% molaire, par exemple plus de 50% molaire, ou plus de 60% molaire, ou plus de 70% molaire, ou plus de 80% molaire, ou encore plus de 90% molaire.

Par définition, la quantité A d'ATBS correspond à la quantité d'ATBS utilisée pour préparer le polymère de l'invention et ayant une concentration en IBSA comprise entre 250 et 20 000 ppm en poids, autrement dit supérieure ou égale à 250 ppm et inférieure ou égale à 20 000 ppm. La quantité B d'ATBS correspond à la quantité d'ATBS utilisée pour préparer le polymère de l'invention et ayant une concentration en IBSA strictement inférieure à 300 ppm, préférentiellement strictement inférieure à 250 ppm, encore plus préférentiellement strictement inférieure à 200 ppm en poids. La quantité totale d'ATBS est la somme des quantités A et B et de la quantité d'ATBS contenant une concentration en IBSA strictement supérieure à 20 000 ppm d'IBSA. Dans un mode préféré, le polymère n'est pas obtenu avec une quantité d'ATBS contenant une concentration en IBSA strictement supérieure à 20 000 ppm d'IBSA.

Dans un mode particulièrement préféré, le polymère est obtenu à partir d'une quantité A d'acide 2-acrylamido-2-méthylpropane sulfonique (forme acide et/ou salifiée) représentant, par rapport à la quantité totale d'ATBS, entre 1 et 100% molaire, préférentiellement plus de 2% molaire, plus préférentiellement plus de 5% molaire, encore plus préférentiellement plus de 10% molaire, encore plus préférentiellement plus de 20% molaire, encore plus préférentiellement plus de 25% molaire, encore plus préférentiellement plus de 30% molaire, encore plus préférentiellement plus de 40% molaire, encore plus préférentiellement plus de 50% molaire, encore plus préférentiellement plus de 60% molaire, encore plus préférentiellement plus de 70% molaire, encore plus préférentiellement plus de 80% molaire, encore plus préférentiellement plus de 90% molaire.

Ainsi, le polymère peut être obtenu au moins à partir d'une quantité A d'ATBS (forme acide et/ou salifiée) et d'une quantité B d'ATBS (forme acide et/ou salifiée), la quantité B contenant strictement moins de 300 ppm, préférentiellement strictement moins de 250 ppm, encore plus préférentiellement strictement moins de 200 ppm en poids d'acide 2-méthyl-2-propényl-sulfonique (IBSA), sous forme acide et/ou salifiée.

Le polymère peut être obtenu au moins à partir d'acide 2-acrylamido-2-méthylpropane sulfonique, sous forme acide et/ou salifiée, dont la quantité totale contient strictement moins de 20 000 ppm en poids d'acide 2-méthyl-2-propenyl-sulfonique, sous forme acide et/ou salifiée.

Dans un autre mode particulièrement préféré concernant les polymères hydrosolubles, la proportion D de la quantité A par rapport à la quantité totale d'acide 2-acrylamido-2-méthylpropane sulfonique, sous forme acide et/ou salifiée, est déterminée à partir du rapport R défini selon l'équation (1) suivante :

$$R = \frac{10^{14}}{[IBSA]^2 * C^2 * Mw} \quad (1)$$

Dans laquelle,

[IBSA] est la concentration, en ppm en poids dans la quantité A, d'acide 2-méthyl-2-propényl-1-sulfonique ;

Mw est le poids moléculaire moyen en poids du polymère exprimé en g/mol ;

C'est un coefficient égal à 0,2 lorsque Mw est inférieur ou égal à 1 million, égal à 0,6 lorsque Mw est strictement supérieur à 1 million et inférieur ou égal à 10 millions, et égal à 0,8 lorsque Mw est strictement supérieur à 10 millions.

Lorsque R est strictement supérieur à 100, D est compris entre 1 et 100%, préférentiellement entre 25 et 95%, plus préférentiellement entre 50 et 90%.

Lorsque R est strictement supérieur à 50 et inférieur ou égal à 100, D est compris entre 1 et 90%, préférentiellement entre 25 et 85%, plus préférentiellement entre 50 et 80%.

Lorsque R est strictement supérieur à 10 et inférieur ou égal à 50, D est compris entre 1 et 80%, préférentiellement entre 25 et 75%, plus préférentiellement entre 50 et 70%.

Lorsque R est strictement supérieur à 5 et inférieur ou égal à 10, D est compris entre 1 et 60%, préférentiellement entre 2 et 50%, plus préférentiellement entre 5 et 40%.

Lorsque R est strictement supérieur à 1 et inférieur ou égal à 5, D est compris entre 1 et 50%, préférentiellement entre 2 et 40%, plus préférentiellement entre 4 et 30%.

Lorsque R est strictement supérieur à 0,1 et inférieur ou égal à 1, D est compris entre 1 et 30%, préférentiellement entre 2 et 25%, plus préférentiellement entre 3 et 20%.

Lorsque R est strictement supérieur à 0,01 et inférieur ou égal à 0,1, D est compris entre 1 et 10%, préférentiellement entre 1 et 8%, plus préférentiellement entre 1 et 6%.

Lorsque R est inférieur ou égal à 0,01, D est compris entre 1 et 5%, préférentiellement entre 1 et 4%, plus préférentiellement entre 1 et 3%.

A titre d'exemple, si pour un homopolymère d'ATBS, la quantité A d'ATBS est de 80 mol% et que la quantité B d'ATBS est de 20 mol%, et que cet homopolymère n'est pas obtenu avec une quantité d'ATBS contenant strictement plus 20 000 pm d'IBSA, alors la proportion D est égale à 80%. Si pour un copolymère d'ATBS et d'acrylamide contenant 50 mol% d'acrylamide, la quantité A d'ATBS est de 30 mol%, la quantité B d'ATBS est de 20 mol% et que ce copolymère n'est pas obtenu avec une quantité d'ATBS contenant strictement plus de 20 000 ppm d'IBSA, alors la proportion D est égale à 60%.

Les quantités d'ATBS peuvent être exprimées en pourcentage molaire ou en pourcentage en poids. Les rapports de ces quantités sont par définition sans unité, par exemple D = A/(A+B) en l'absence d'ATBS contenant strictement plus 20 000 pm d'IBSA.

Dans un mode de réalisation particulier, le polymère peut être obtenu avec une quantité « a », contenant par exemple 280 ppm d'IBSA et une quantité « a' » contenant par exemple 560 ppm d'IBSA. La quantité A sera la somme des quantités « a » et « a' » et la concentration d'IBSA pourra facilement être déterminée en fonction de la proportion des deux quantités « a » et « a' ».

Selon un mode préféré de l'invention, le polymère de l'invention est obtenu à partir de la forme salifiée de l'acide 2-acrylamido-2-méthylpropane sulfonique. L'acide 2-acrylamido-2-méthylpropane sulfonique est donc de préférence partiellement ou totalement salifié avant polymérisation. La forme acide d'un monomère peut être salifiée avant et/ou pendant et/ou après la polymérisation des monomères.

La forme salifiée est avantageusement obtenue à partir d'un composé choisi parmi un hydroxyde de métal alcalin ou alcalino-terreux, un oxyde de métal alcalin ou alcalino-terreux, l'ammoniaque, une amine de formule suivante $NR_1R_2R_3$ ($R_1$, $R_2$ et $R_3$, identiques ou différents, étant avantageusement des groupements hydrocarbonés, notamment des alkyles) et un carbonate de métal alcalin ou alcalino-terreux. Un métal alcalin préféré est le sodium.

Comme précédemment exposé, le polymère de l'invention peut être un copolymère obtenu à partir d'acide 2-acrylamido-2-méthylpropane sulfonique (forme acide et/ou salifiée) et d'au moins un monomère non ionique, et/ou au moins un monomère anionique, et/ou au moins un monomère cationique et/ou au moins un monomère zwittérionique.

Les monomères non ioniques peuvent être choisis, notamment, dans le groupe comprenant les monomères vinyliques solubles dans l'eau. Des monomères préférés appartenant à cette classe sont, par exemple, l'acrylamide, le méthacrylamide, le N-isopropylacrylamide, le N,N-diméthylacrylamide, le N,N-diéthylacrylamide et le N-méthylolacrylamide. Egalement, peuvent être utilisés la N-vinylformamide (NVF), le N-vinyl acétamide, la N-vinylpyridine et la N-vinylpyrrolidone (NVP), le N-vinyl imidazole, le N-vinyl succinimide, l'acryloyl morpholine (ACMO), le chlorure d'acryloyl, le méthacrylate de glycidyle, le méthacrylate de glycéryle, la diacétone acrylamide et l'isoprénol. Un monomère non ionique préféré est l'acrylamide.

Les monomères anioniques peuvent être choisis dans un large groupe. Ces monomères peuvent présenter des fonctions vinyliques, notamment acryliques, maléiques, fumariques, malonique, itaconique, ou allyliques, et contenir un groupe carboxylate, phosphonate, phosphate, sulfate, sulfonate, ou un autre groupe à charge anionique. Le monomère anionique peut être sous forme acide ou bien sous forme salifiée, par exemple un sel de métal alcalino-terreux, un sel de métal alcalin ou un sel d'ammonium. Des exemples de monomères convenables incluent l'acide acrylique ; l'acide méthacrylique ; l'acide itaconique ; l'acide crotonique ; l'acide maléique ; l'acide fumarique ; l'acide acrylamido undécanoïque ; l'acide 3-acrylamido 3-méthylbutanoïque ; l'anhydride maléique ; les monomères de type acide fort présentant par exemple une fonction de type acide sulfonique ou acide phosphonique tels que l'acide vinylsulfonique, l'acide vinylphosphonique, l'acide allylsulfonique, l'acide méthallylsulfonique, le 2-sulfoéthylméthacrylate, le sulfopropylméthacrylate, le sulfopropylacrylate, l'acide allylphosphonique, l'acide styrène sulfonique, l'acide 2-acrylamido-2-méthylpropane disulfonique ; et les sels hydrosolubles de ces monomères comme leurs sels de métaux alcalins, de métaux alcalino-terreux, ou d'ammonium. Dans cette liste, les monomères mentionnés de type acide fort présentant une fonction de type acide sulfonique n'incluent pas la quantité A d'acide 2-acrylamido-2-méthylpropane sulfonique. Par contre, ils incluent la quantité B d'acide 2-acrylamido-2-méthylpropane sulfonique purifié comprenant strictement moins de 300 ppm, préférentiellement strictement moins de 250 ppm, encore plus préférentiellement strictement moins de 200 ppm d'IBSA. De préférence, ils excluent toute quantité d'ATBS contenant strictement plus de 20 000 ppm en poids d'IBSA.

Les monomères cationiques peuvent être choisis, notamment parmi les monomères dérivés des motifs de type vinylique, notamment acrylamide, acrylique, allylique ou maléique, ces monomères possédant une fonction phosphonium ou ammonium. L'ammonium est avantageusement tertiaire ou quaternaire, plus avantageusement quaternaire. On peut citer, en particulier et de façon non limitative, l'acrylate de diméthylaminoéthyle (ADAME) quaternisé, le méthacrylate de diméthylaminoéthyle (MADAME) quaternisé, le chlorure de diméthyldiallylammonium (DADMAC), le chlorure d'acrylamido propyltriméthyl ammonium (APTAC), et le chlorure de méthacrylamido propyltriméthyl ammonium (MAPTAC).

Les sels acidifiés sont obtenus par les moyens connus de la personne du métier, et notamment par protonation. Les sels quaternisés sont également obtenus par les moyens connus de la personne du métier notamment, par réaction avec le chlorure de benzyle, le chlorure de méthyle (MeCl), les chlorures d'aryle, d'alkyle, ou les dialkylsulfates comme le diméthylsulfate. L'agent de quaternisation peut être choisi parmi les chlorures d'alkyle, les sulfates de dialkyle et les halogénures d'alkyle. Préférentiellement, l'agent de quaternisation est choisi parmi le chlorure de méthyle et le diéthyle sulfate.

Le monomère zwitterionique peut être un dérivé d'un motif de type vinylique, notamment acrylamide, acrylique, allylique ou maléique, ce monomère possédant une fonction cationique (avantageusement ammonium quaternaire) et une fonction anionique (avantageusement acide de type carboxylique ou carboxylate), sulfonique (ou sulfonate) ou phosphorique (ou phosphate). On peut citer, en particulier et de façon non limitative, les dérivés de l'acrylate de diméthylaminoéthyl, tel que le 2-((2-(acryloyloxy)éthyl) diméthylammonio) éthane-1-sulfonate, le 3-((2-(acryloyloxy)éthyl) diméthylammonio) propane-1-sulfonate, le 4-((2-(acryloyloxy)éthyl) diméthylammonio) butane-1-sulfonate, le [2-(acryloyloxy)éthyl] (diméthylammonio) acétate, les dérivés du méthacrylate de diméthylaminoéthyle tel que le 2-((2-(méthacryloyloxy) éthyl) diméthylammonio) éthane-1-sulfonate, le 3-((2-(méthacryloyloxy) éthyl) diméthylammonio) propane-1-sulfonate, le 4-((2-(méthacryloyloxy) éthyl) diméthylammonio) butane-1-sulfonate, le [2-(méthacryloyloxy)éthyl] (diméthylammonio) acétate, les dérivés du diméthylamino propylacrylamide tel que le 2-((3-acrylamidopropyl) diméthylammonio) éthane-1-sulfonate, le 3-((3-acrylamidopropyl) diméthylammonio) propane-1-sulfonate, le 4-((3-acrylamidopropyl) diméthylammonio) butane-1-sulfonate, le [3-(acryloyloxy) propyl] (diméthylammonio) acétate, les dérivés du diméthylamino propyl méthylacrylamide tel que le 2-((3-méthacrylamidopropyl) diméthylammonio) éthane-1-sulfonate, le 3-((3-méthacrylamidopropyl) diméthylammonio) propane-1-sulfonate, le 4-((3-méthacrylamidopropyl) diméthylammonio) butane-1-sulfonate et le [3-(méthacryloyloxy)propyl] (diméthylammonio) acétate.

La quantité totale de monomères non ioniques, anioniques, cationiques ou zwitterioniques des listes précédentes peut être comprise entre 1 et 99% molaire, généralement entre 10 et 80%, plus généralement entre 20 et 50% molaire. La personne de métier saura ajuster la proportion de chaque monomère pour que la somme des monomères représente 100% molaire, incluant l'ATBS. Sauf indication contraire, les pourcentages sont des pourcentages molaires exprimé par rapport au nombre total de tous les monomères, incluant l'ATBS.

Les monomères présentant un caractère hydrophobe peuvent également être utilisés dans la préparation du polymère de l'invention. Ils sont de préférence choisis dans le groupe constitué par les esters de l'acide (méth)acrylique

présentant une chaîne alkyle, arylalkyle, propoxylée, éthoxylée, ou éthoxylée et propoxylée ; les dérivés du (méth)acrylamide présentant une chaîne alkyle, arylalkyle propoxylée, éthoxylée, éthoxylée et propoxylée, ou dialkyle ; les alkyl aryl sulfonates.

Lorsqu'on utilise un monomère présentant un caractère hydrophobe pour la préparation du (co)polymère hydrosoluble, sa quantité se situe avantageusement dans la plage comprise entre 0,001 et 3 % molaire.

Dans un mode préféré, les polymères selon l'invention ne contiennent pas de monomère acrylonitrile ou de ses dérivés, ni de monomère styrène ou de ses dérivés.

Selon l'invention, le polymère peut avoir une structure linéaire, ramifiée, star (en forme d'étoile), comb (en forme de peigne), dendritique ou bloc. Ces structures peuvent être obtenues par sélection au choix de l'amorceur, de l'agent de transfert, de la technique de polymérisation (polymérisation radicalaire contrôlée dite RAFT (transfert de chaîne réversible par addition-fragmentation), de l'anglais « réversible-addition fragmentation chain transfer »), NMP (polymérisation en présence de nitroxydes, de l'anglais « Nitroxide Mediated Polymerization ») ou ATRP (polymérisation radicalaire par transfert d'atomes, de l'anglais « Atom Transfert Radical Polymerization »), de l'incorporation de monomères structuraux, ou de la concentration en monomère(s).

Selon l'invention, le polymère est avantageusement linéaire ou structuré. Par polymère structuré, on désigne un polymère non linéaire qui possède des chaînes latérales de manière à obtenir, lorsque ce polymère est mis en solution dans l'eau, un fort état d'enchevêtrement conduisant à des viscosités à bas gradient très importantes.

Le polymère peut en outre être structuré :

- par au moins un agent de structure, pouvant notamment être choisi dans le groupe comprenant des monomères à insaturation polyéthylénique (ayant au minimum deux fonctions insaturées), comme par exemple les fonctions vinyliques, allyliques, acryliques et époxy et l'on peut citer par exemple le méthylène bis acrylamide (MBA), la triallyamine, ou le chlorure de tétraallylammonium ou 1,2 dihydroxyéthylène bis-(N-acrylamide), et/ou
- par des macroamorceurs tels que les polyperoxydes, polyazoïques et les polyagents de transfert tels que les (co)polymères polymercaptans, et les polyols, et/ou
- par des polysaccharides fonctionnalisés.

La quantité d'agent de de structure, avantageusement un agent de ramification/réticulation, dans le mélange de monomères est avantageusement inférieure à 4 % en poids par rapport à la quantité de monomère(s), plus avantageusement inférieure à 1 %, et encore plus avantageusement inférieure à 0,5 %. Selon un mode de réalisation particulier, elle peut être au moins égale à 0,00001 % en poids par rapport à la quantité de monomère(s).

De manière générale, le polymère ne nécessite pas de développement de procédé de polymérisation particulier. En effet, il peut être obtenu selon toutes les techniques de polymérisation bien connues par la personne de métier. Il peut notamment s'agir de polymérisation en solution ; polymérisation en gel ; polymérisation par précipitation ; polymérisation en émulsion (aqueuse ou inverse) ; polymérisation en suspension ; polymérisation par extrusion réactive ; polymérisation eau dans eau ; ou de polymérisation micellaire.

La polymérisation est généralement une polymérisation à radicaux libres, de préférence par polymérisation en émulsion inverse ou par polymérisation en gel. Par polymérisation par radicaux libres, sont incluses la polymérisation par radicaux libres au moyen d'initiateurs UV, azoïques, redox ou thermiques ainsi que les techniques de polymérisation radicalaire contrôlée (CRP) et les techniques de polymérisation sur matrice.

L'invention a également pour objet l'utilisation du polymère selon l'invention dans la récupération du pétrole et du gaz, dans le traitement de l'eau, dans le traitement des boues, dans la fabrication du papier, dans la construction, dans l'industrie minière, dans la formulation de produits cosmétiques, dans la formulation de détergents, dans la fabrication du textile, ou dans l'agriculture. L'invention a aussi pour objet l'utilisation dudit polymère comme floculant, agent épaississant, agent absorbant ou agent réducteur de friction. Dans la fabrication du papier, le polymère de l'invention peut avantageusement être utilisé pour améliorer la résistance à sec du papier. Dans la construction, le polymère de l'invention peut être avantageusement utilisé comme plastifiant ou superplastifiant.

L'invention a aussi pour objet l'utilisation du polymère selon l'invention comme floculant, agent réducteur de viscosité, agent épaississant, agent absorbant, agent réducteur de friction, agent plastifiant ou superplastifiant.

De manière tout à fait surprenante il est ainsi possible de polymériser un monomère d'acide 2-acrylamido-2-méthylpropane sulfonique considéré comme étant de basse qualité (250-20 000 ppm, avantageusement 300-20 000 ppm en poids d'IBSA) et d'obtenir un polymère de haut poids moléculaire sans que les caractéristiques du polymère ne soient substantiellement affectées.

Autrement dit, là où il semblait nécessaire de purifier l'acide 2-acrylamido-2-méthylpropane sulfonique, l'invention démontre qu'il est possible de réduire le niveau d'exigence lors d'une étape de purification, et idéalement de se passer d'étape de purification de l'acide 2-acrylamido-2-méthylpropane sulfonique et d'obtenir des polymères de haut poids moléculaire de performances satisfaisantes.

Les exemples suivants ne sont donnés qu'à titre d'illustration de l'objet de l'invention, sans la limiter en aucune

manière.

**Exemples**

**Exemple 1 (comparatif) : Préparation d'un homopolymère d'acide 2-acrylamido-2-methylpropane sulfonique.**

Dans un bécher de 2000 mL, sont ajoutés 390,5 g d'eau déionisée, 262 g de lessive de soude à 50 % (en poids dans l'eau) et 847,5 g de cristaux d'acide 2-acrylamido-2-méthylpropane sulfonique contenant 132 ppm d'IBSA.
La solution ainsi obtenue est refroidie entre 5 et 10°C et transférée dans un réacteur de polymérisation adiabatique, un bullage d'azote est réalisé pendant 30 minutes afin d'éliminer toute trace d'oxygène dissous.
Sont ensuite ajoutés dans le réacteur :

- 0,45 g de 2,2'-azobisisobutyronitrile,
- 1,5 mL d'une solution aqueuse à 2,5 g/L de 2,2'-Azobis[2-(2-imidazolin-2-yl)propane] dihydrochlorure,
- 1,5 mL d'une solution aqueuse à 1 g/L d'hypophosphite de sodium,
- 1,5 mL d'une solution aqueuse à 1 g/L de tert-butyl hydroperoxyde,
- 1,5 mL d'une solution aqueuse à 1 g/L de sulfate d'ammonium et de fer(II) hexahydraté (sel de Mohr).

Après quelques minutes, l'arrivée d'azote est fermée et le réacteur est clos. La réaction de polymérisation se déroule pendant 4 heures pour atteindre un pic de température. Le gel caoutchouteux obtenu est haché et séché pour obtenir une poudre grossière elle-même broyée et tamisée pour obtenir l'homopolymère d'acide 2-acrylamido-2-methylpropane sulfonique sous forme de poudre.

**Exemple 2 (comparatif) : Préparation d'un copolymère acrylamide/acide 2-acrylamido-2-methylpropane sulfonique (75/25 mol%)**

Dans un bécher de 2000 mL sont ajoutés 549,55 g d'eau déionisé, 520,5g d'acrylamide en solution à 50% (en poids dans l'eau), 97,6 g de lessive de soude à 50 % (en poids dans l'eau) et 316,2 g de cristaux d'acide 2-acrylamido-2-méthylpropane sulfonique contenant 132 ppm d'IBSA. Les mêmes procédures de polymérisation et de traitement du gel que dans l'exemple 1 sont effectuées. On obtient un polymère sous forme de poudre.

**Exemple 3 (comparatif) : Préparation d'un copolymère acrylamide/acide 2-acrylamido-2-methylpropane sulfonique (75/25 mol%)**

La même préparation que dans l'exemple 2 est réalisée, à la seule différence près que 0,5 mL d'une solution aqueuse à 1 g/L d'hypophosphite de sodium sont utilisés au lieu de 1,5 mL. On obtient un polymère sous forme de poudre.

**Exemple 4 (invention) :**

La même préparation que dans l'exemple 1 est réalisée à la seule différence près que 53% en poids de l'acide 2-acrylamido-2-methylpropane sulfonique, soit 449,175g, sont substitués par de l'acide 2-acrylamido-2-methylpropane sulfonique contenant 1042 ppm d'IBSA. La proportion D est de 53%.

**Exemple 5 (invention) :**

La même préparation que dans l'exemple 2 est réalisée à la seule différence près que 55% de l'acide 2-acrylamido-2-methylpropane sulfonique, soit 173,91g, sont substitués par de l'acide 2-acrylamido-2-methylpropane sulfonique contenant 715 ppm en poids d'IBSA. La proportion D est de 55%.

**Exemple 6 (invention)** :

La même préparation que dans l'exemple 3 est réalisée à la seule différence près que 23% de l'acide 2-acrylamido-2-methylpropane sulfonique, soit 72,726g, sont substitués par de l'acide 2-acrylamido-2-methylpropane sulfonique contenant 1210 ppm en poids d'IBSA. La proportion D est de 23%.

**Exemple 7 (invention)** :

La même préparation que dans l'exemple 1 est réalisée à la seule différence près que 58% en poids de l'acide

2-acrylamido-2-methylpropane sulfonique, soit 491,55g, sont substitués par de l'acide 2-acrylamido-2-methylpropane sulfonique contenant 1042 ppm d'IBSA. La proportion D est de 58%.

**Exemple 8 (invention)** :

La même préparation que dans l'exemple 2 est réalisée à la seule différence près que 60% de l'acide 2-acrylamido-2-methylpropane sulfonique, soit 189,72g, sont substitués par de l'acide 2-acrylamido-2-methylpropane sulfonique contenant 715 ppm en poids d'acide 2-methyl-2-propenyl-1-sulfonique. La proportion D est de 60%.

**Exemple 9 (invention)** :

La même préparation que dans l'exemple 3 est réalisée à la seule différence près que 30% de l'acide 2-acrylamido-2-methylpropane sulfonique, soit 94,86g, sont substitués par de l'acide 2-acrylamido-2-methylpropane sulfonique contenant 1210 ppm en poids d'IBSA. La proportion D est de 30%.

Le poids moléculaire moyen en poids des polymères des exemples 1 à 9 est mesuré selon la méthode exposée précédemment, et les résultats sont exposés dans le tableau 1.

Tableau 1 - Poids moléculaire moyen en poids des polymères 1 à 9, rapport R et proportion D des polymères 4 à 9.

| Exemple | Poids moléculaire moyen en poids (en millions de g/mol) | C | [IBSA] (ppm par rapport à la quantité A) | R | Proportion D |
|---|---|---|---|---|---|
| 1 | 1,12 | - | - | - | |
| 2 | 6,78 | - | - | - | |
| 3 | 11,23 | - | - | - | |
| 4 | 1,10 | 0,60 | 1042 | 232.6 | 53% |
| 5 | 6,81 | 0,60 | 715 | 79.8 | 55% |
| 6 | 11,22 | 0,80 | 1210 | 9.5 | 23% |
| 7 | 1,05 | 0,60 | 1042 | 243.7 | 58% |
| 8 | 6,72 | 0,60 | 715 | 80.9 | 60% |
| 9 | 11,08 | 0,80 | 1210 | 9.6 | 30% |

Ainsi, la substitution d'une partie de l'ATBS de bonne pureté (< 200 ppm d'IBSA) par de l'ATBS de mauvaise pureté (250-20 000 ppm d'IBSA) n'impacte pas significativement le poids moléculaire des polymères.

**Exemple 10 - Test applicatif 1**

Les polymères 2, 3, 5, 6, 8 et 9 sont dissous dans de l'eau du robinet afin d'obtenir des solutions aqueuses présentant une concentration à 0,1% en poids du polymère par rapport au poids total de la solution. Les solutions sont agitées mécaniquement à 200 t/min jusqu'à la solubilisation complète des polymères et l'obtention de solutions claires et homogènes.

Une série de tests de floculation est effectuée sur un effluent aqueux contenant 30 g/L de Kaolin, 1 g/L de chlorure de calcium et 100 g/L de minerai broyé.

Les tests sont réalisés en Jar Test manuel selon le protocole suivant :

- Remplissage des tubes avec l'effluent ;
- Injection d'une solution polymérique à différents dosages ;
- Réalisation de 5 renversements du Jar Test pour incorporation de la solution aqueuse polymérique dans la suspension de l'effluent.

Les résultats présentés dans le tableau 2 récapitulent la vitesse de sédimentation selon le dosage de polymère mis en oeuvre par rapport à la quantité d'effluent.

Tableau 2 Résultat des tests de floculation

| **Exemple** | **Dosage polymère (g/tonne)** | | | |
|---|---|---|---|---|
| | 4 | 8 | 12 | 16 |
| | **Vitesse de sédimentation (m/h)** | | | |
| 2 | 10 | 22 | 41 | 65 |
| 3 | 12 | 24 | 41 | 63 |
| 5 | 11 | 23 | 42 | 64 |
| 6 | 10 | 22 | 43 | 64 |
| 8 | 10 | 22 | 42 | 62 |
| 9 | 9 | 21 | 41 | 62 |

Ainsi, la substitution d'une partie de l'ATBS de bonne pureté (< 250 ppm d'IBSA) par de l'ATBS de mauvaise pureté (250-20 000 ppm d'IBSA) n'impacte pas significativement les performances des polymères.

**Exemple 11 : Test applicatif 2**

Des solutions des polymères 1, 4 et 7 sont préparées à une concentration active de 1 000 ppm en poids dans une saumure contenant de l'eau, 30 000 ppm en poids de NaCl et 3 000 ppm en poids de $CaCl_2,2H_2O$. Les polymères sont testés dans le cadre d'une application de récupération assistée du pétrole par injection de solutions polymériques. Le quotient de filtration (FR) est mesuré sur des filtres ayant une taille de pore de 1,2 $\mu$m représentatifs de gisements de faible perméabilité.

Le terme quotient de filtration (ou filter ratio noté « FR ») est utilisé dans le présent document pour désigner un test utilisé pour déterminer la performance de la solution de polymère dans des conditions approchant la perméabilité du gisement consistant à mesurer le temps pris par des volumes/concentrations donnés de solution pour traverser un filtre. Le FR compare généralement la filtrabilité de la solution polymère pour deux volumes consécutifs équivalents, ce qui indique la tendance de la solution à boucher le filtre. Les FR plus faibles indiquent un meilleur rendement.

Le test utilisé pour déterminer le FR consiste à mesurer les temps que mettent des volumes donnés de solution à 1000 ppm actif de polymère pour s'écouler au travers d'un filtre. La solution est contenue dans une cellule pressurisée à deux bars de pression et le filtre est de 47 mm de diamètre et d'une taille de pores définies. Généralement, le FR est mesuré avec des filtres ayant une taille de pores de 1,2 $\mu$m, 3 $\mu$m, 5 $\mu$m ou 10 $\mu$m. Dans l'exemple, la taille des pores est de 1,2$\mu$m.

Les temps nécessaires pour obtenir 100 mL ($t_{100mL}$) ; 200 mL ($t_{200mL}$) et 300 mL ($t_{300mL}$) de filtrat sont donc mesurés. Le quotient de filtration FR est défini par :

$$FR = \frac{t_{300\,mL} - t_{200\,mL}}{t_{200\,mL} - t_{100\,mL}}$$

Les temps sont mesurés à 0,1 seconde près.

Le FR représente ainsi la capacité de la solution de polymère à colmater le filtre pour deux volumes consécutifs équivalents.

Les résultats sont présentés dans le tableau 3 suivant.

Tableau 3 - Résultat de FR

| **Exemple** | **FR** |
|---|---|
| 1 | 1,16 |
| 4 | 1,16 |
| 7 | 1,17 |

Ainsi, la substitution d'une partie de l'ATBS de bonne pureté (< 300 ppm d'IBSA, voire < 250 ou < 200 ppm d'IBSA) par de l'ATBS de mauvaise pureté (250-20 000 ppm d'IBSA ou 300-20 000) n'impacte pas significativement

les performances des polymères.

## Revendications

1. Polymère obtenu au moins à partir d'une quantité A d'acide 2-acrylamido-2-méthylpropane sulfonique, sous forme acide et/ou salifiée, ***caractérisé* en ce que** la quantité A d'acide 2-acrylamido-2-méthylpropane sulfonique contient de 250 à 20 000 ppm en poids d'acide 2-méthyl-2-propényl-sulfonique, sous forme acide et/ou salifiée et entre 300 et 10 000 ppm en poids d'acide 2-méthylidène-1,3-propylènedisulfonique.

2. Polymère selon la revendication 1, ***caractérisé* en ce que** la quantité A d'acide 2-acrylamido-2-méthylpropane sulfonique, sous forme acide et/ou salifiée, contient 300 à 20 000 ppm d'acide 2-méthyl-2-propényl-sulfonique, sous forme acide et/ou salifiée.

3. Polymère selon la revendication 1, ***caractérisé* en ce que** la quantité A d'acide 2-acrylamido-2-méthylpropane sulfonique, sous forme acide et/ou salifiée, contient 300 à 10 000 ppm d'acide 2-méthyl-2-propényl-sulfonique, sous forme acide et/ou salifiée.

4. Polymère selon l'une des revendications 1 à 3, ***caractérisé* en ce que** le polymère est obtenu en outre à partir d'une quantité B d'acide 2-acrylamido-2-méthylpropane sulfonique, sous forme acide et/ou salifiée, contenant strictement moins de 200 ppm en poids d'acide 2-méthyl-2-propényl-sulfonique, sous forme acide et/ou salifiée.

5. Polymère selon l'une des revendications 1 à 4, ***caractérisé* en ce que** le polymère est un polymère hydrosoluble ou un polymère superabsorbant.

6. Polymère selon l'une des revendications 1 à 5, ***caractérisé* en ce que** le polymère est hydrosoluble et présente un poids moléculaire moyen en poids supérieur à 100 000 g/mol et inférieur ou égal à 40 millions de g/mol, le poids moléculaire moyen étant déterminé par la viscosité intrinsèque du polymère.

7. Polymère selon l'une des revendications 1 à 6, ***caractérisé* en ce que** le polymère est un homopolymère d'acide 2-acrylamido-2-méthylpropane sulfonique, sous forme acide et/ou salifiée, ou un copolymère d'acide 2-acrylamido-2-méthylpropane sulfonique, sous forme acide et/ou salifiée, comprenant en outre au moins un monomère choisi dans le groupe comprenant les monomères non ioniques, les monomères anioniques, les monomères cationiques, les monomères zwitterionique, et leurs mélanges.

8. Polymère selon l'une des revendications 1 à 7, ***caractérisé* en ce que** le polymère est obtenu au moins à partir d'acide 2-acrylamido-2-méthylpropane sulfonique, sous forme acide et/ou salifiée, dont la quantité totale contient strictement moins de 20 000 ppm en poids d'acide 2-méthyl-2-propenyl-sulfonique, sous forme acide et/ou salifiée.

9. Polymère selon l'une des revendications 1 à 8, ***caractérisé* en ce que** le polymère est hydrosoluble et **en ce que** la proportion D de la quantité A par rapport à la quantité totale d'acide 2-acrylamido-2-méthylpropane sulfonique, sous forme acide et/ou salifiée, est déterminée à partir du rapport R défini selon l'équation (1) suivante :

$$R = \frac{10^{14}}{[IBSA]^2 * C^2 * Mw} \quad (1)$$

Dans laquelle,
[IBSA] est la concentration en ppm en poids dans la quantité A d'acide 2-méthyl-2-propényl-1-sulfonique ;
Mw est le poids moléculaire moyen en poids du polymère exprimé en g/mol ;
C est un coefficient égal à 0,2 lorsque Mw est inférieur ou égal à 1 million, égal à 0,6 lorsque Mw est strictement supérieur à 1 million et inférieur ou égal à 10 millions, et égal à 0,8 lorsque Mw est strictement supérieur à 10 millions ;
Lorsque R est strictement supérieur à 100, D est compris entre 1 et 100% ;
Lorsque R est strictement supérieur à 50 et inférieur ou égal à 100, D est compris entre 1 et 90% ;
Lorsque R est strictement supérieur à 10 et inférieur ou égal à 50, D est compris entre 1 et 80% ;

Lorsque R est strictement supérieur à 5 et inférieur ou égal à 10, D est compris entre 1 et 60% ;
Lorsque R est strictement supérieur à 1 et inférieur ou égal à 5, D est compris entre 1 et 50% ;
Lorsque R est strictement supérieur à 0,1 et inférieur ou égal à 1, D est compris entre 1 et 30% ;
Lorsque R est strictement supérieur à 0,01 et inférieur ou égal à 0,1, D est compris entre 1 et 10% ;
Lorsque R est inférieur ou égal à 0,01, D est compris entre 1 et 5%.

**10.** Polymère selon la revendication 9, ***caractérisé* en ce que**

Lorsque R est strictement supérieur à 100, D est compris entre 25 et 95% ;
Lorsque R est strictement supérieur à 50 et inférieur ou égal à 100, D est compris entre 25 et 85% ;
Lorsque R est strictement supérieur à 10 et inférieur ou égal à 50, D est compris entre 25 et 75% ;
Lorsque R est strictement supérieur à 5 et inférieur ou égal à 10, D est compris entre 2 et 50% ;
Lorsque R est strictement supérieur à 1 et inférieur ou égal à 5, D est compris entre 2 et 40% ;
Lorsque R est strictement supérieur à 0,1 et inférieur ou égal à 1, D est compris entre 2 et 25% ;
Lorsque R est strictement supérieur à 0,01 et inférieur ou égal à 0,1, D est compris entre 1 et 8%;
Lorsque R est inférieur ou égal à 0,01, D est compris entre 1 et 4%.

**11.** Polymère selon la revendication 9, ***caractérisé* en ce que**

Lorsque R est strictement supérieur à 100, D est compris entre 50 et 90% ;
Lorsque R est strictement supérieur à 50 et inférieur ou égal à 100, D est compris entre 50 et 80% ;
Lorsque R est strictement supérieur à 10 et inférieur ou égal à 50, D est compris entre 50 et 70% ;
Lorsque R est strictement supérieur à 5 et inférieur ou égal à 10, D est compris entre 5 et 40% ;
Lorsque R est strictement supérieur à 1 et inférieur ou égal à 5, D est compris entre 4 et 30% ;
Lorsque R est strictement supérieur à 0,1 et inférieur ou égal à 1, D est compris entre 3 et 20% ;
Lorsque R est strictement supérieur à 0,01 et inférieur ou égal à 0,1, D est compris entre 1 et 6%;
Lorsque R est inférieur ou égal à 0,01, D est compris entre 1 et 3%.

**12.** Utilisation du polymère selon l'une des revendications 1 à 11 dans un domaine choisi parmi la récupération du pétrole et du gaz, le traitement de l'eau, le traitement des boues, la fabrication du papier, la construction, l'industrie minière, la formulation de produits cosmétiques, la formulation de détergents, la fabrication du textile, et l'agriculture.

**13.** Utilisation du polymère selon l'une des revendications 1 à 12 comme floculant, agent réducteur de viscosité, agent épaississant, agent absorbant, agent réducteur de friction ou agent plastifiant ou superplastifiant.

## Patentansprüche

**1.** Polymer, das mindestens aus einer Menge A an 2-Acrylamido-2-methylpropansulfonsäure in Säure- und/oder Salzform erhalten wird, ***dadurch gekennzeichnet,* dass** die Menge A an 2-Acrylamido-2-methylpropansulfonsäure 250 bis 20.000 Gew.-ppm 2-Methyl-2-propenyl-sulfonsäure in Säure- und/oder Salzform und 300 bis 10.000 Gew.-ppm 2-Methyliden-1,3-propylendisulfonsäure enthält.

**2.** Polymer nach Anspruch 1, ***dadurch gekennzeichnet,* dass** die Menge A an 2-Acrylamido-2-methylpropansulfonsäure, in Säure- und/oder Salzform, 300 bis 20.000 ppm 2-Methyl-2-propenyl-sulfonsäure, in Säure- und/oder Salzform, enthält.

**3.** Polymer nach Anspruch 1, ***dadurch gekennzeichnet,* dass** die Menge A an 2-Acrylamido-2-methylpropansulfonsäure, in Säure- und/oder Salzform, 300 bis 10.000 ppm 2-Methyl-2-propenyl-sulfonsäure, in Säure- und/oder Salzform, enthält.

**4.** Polymer nach einem der Ansprüche 1 bis 3, ***dadurch gekennzeichnet,* dass** das Polymer zusätzlich aus einer Menge B an 2-Acrylamido-2-methylpropansulfonsäure in Säure- und/oder Salzform erhalten wird, die strikt weniger als 200 Gew.-ppm 2-Methyl-2-propenyl-sulfonsäure in Säure- und/oder Salzform enthält.

**5.** Polymer nach einem der Ansprüche 1 bis 4, ***dadurch gekennzeichnet,* dass** das Polymer ein wasserlösliches Polymer oder ein superabsorbierendes Polymer ist.

6. Polymer nach einem der Ansprüche 1 bis 5, ***dadurch gekennzeichnet,*** **dass** das Polymer wasserlöslich ist und ein gewichtsmittleres Molekulargewicht von mehr als 100.000 g/mol und weniger als oder gleich 40 Millionen g/mol aufweist, wobei das gewichtsmittlere Molekulargewicht durch die intrinsische Viskosität des Polymers bestimmt wird.

7. Polymer nach einem der Ansprüche 1 bis 6, ***dadurch gekennzeichnet,*** **dass** das Polymer ein Homopolymer von 2-Acrylamido-2-methylpropansulfonsäure, in Säure- und/oder Salzform, oder ein Copolymer von 2-Acrylamido-2-methylpropansulfonsäure, in Säure- und/oder Salzform, ist, das ferner mindestens ein Monomer umfasst, das aus der Gruppe ausgewählt ist, die aus nichtionischen Monomeren, anionischen Monomeren, kationischen Monomeren, zwitterionischen Monomeren und Gemischen davon besteht.

8. Polymer nach einem der Ansprüche 1 bis 7, ***dadurch gekennzeichnet,*** **dass** das Polymer zumindest aus 2-Acrylamido-2-methylpropansulfonsäure in Säure- und/oder Salzform erhalten wird, deren Gesamtmenge strikt weniger als 20.000 Gew.-ppm 2-Methyl-2-propenyl-sulfonsäure in Säure- und/oder Salzform enthält.

9. Polymer nach einem der Ansprüche 1 bis 8, ***dadurch gekennzeichnet,*** **dass** das Polymer wasserlöslich ist und dass das Verhältnis D der Menge A zur Gesamtmenge an 2-Acrylamido-2-methylpropansulfonsäure, in Säure- und/oder Salzform, nach dem Verhältnis R in folgender Gleichung (1) bestimmt wird:

$$R = \frac{10^{14}}{[IBSA]^2 * C^2 * Mw} \quad (1)$$

In welcher
[IBSA] die Konzentration in Gewichts-ppm in der Menge A von 2-Methyl-2-propenyl-l-sulfonsäure ist;
Mw das mittlere Molekulargewicht des Polymers in g/mol ist;
C ein Koeffizient gleich 0,2 ist, wenn Mw kleiner oder gleich 1 Million ist, gleich 0,6, wenn Mw strikt größer als 1 Million und kleiner oder gleich 10 Millionen ist, und gleich 0,8, wenn Mw strikt größer als 10 Millionen ist;
Wenn R strikt größer als 100 ist, liegt D zwischen 1 und 100 %;
Wenn R strikt größer als 50 und kleiner oder gleich 100 ist, liegt D zwischen 1 und 90 %;
Wenn R strikt größer als 10 und kleiner oder gleich 50 ist, liegt D zwischen 1 und 80 %;
Wenn R strikt größer als 5 und kleiner oder gleich 10 ist, liegt D zwischen 1 und 60 %;
Wenn R strikt größer als 1 und kleiner oder gleich 5 ist, liegt D zwischen 1 und 50 %;
Wenn R strikt größer als 0,1 und kleiner oder gleich 1 ist, liegt D zwischen 1 und 30 %;
Wenn R strikt größer als 0,01 und kleiner als oder gleich 0,1 ist, liegt D zwischen 1 und 10 %;
Wenn R kleiner als oder gleich 0,01 ist, liegt D zwischen 1 und 5 %.

10. Polymer nach Anspruch 9, das wie folgt ***gekennzeichnet*** ist:

Wenn R strikt größer als 100 ist, liegt D zwischen 25 und 95 %,
Wenn R strikt größer als 50 und kleiner oder gleich 100 ist, liegt D zwischen 25 und 85 %;
Wenn R strikt größer als 10 und kleiner oder gleich 50 ist, liegt D zwischen 25 und 75 %;
Wenn R strikt größer als 5 und kleiner oder gleich 10 ist, liegt D zwischen 2 und 50 %;
Wenn R strikt größer als 1 und kleiner oder gleich 5 ist, liegt D zwischen 2 und 40 %;
Wenn R strikt größer als 0,1 und kleiner oder gleich 1 ist, liegt D zwischen 2 und 25 %;
Wenn R strikt größer als 0,01 und kleiner als oder gleich 0,1 ist, liegt D zwischen 1 und 8%;
Wenn R kleiner oder gleich 0,01 ist, liegt D zwischen 1 und 4 %.

11. Polymer nach Anspruch 9, das wie folgt ***gekennzeichnet*** ist:

Wenn R strikt größer als 100 ist, liegt D zwischen 50 und 90 %;
Wenn R strikt größer als 50 und kleiner oder gleich 100 ist, liegt D zwischen 50 und 80 %;
Wenn R strikt größer als 10 und kleiner oder gleich 50 ist, liegt D zwischen 50 und 70 %;
Wenn R strikt größer als 5 und kleiner oder gleich 10 ist, liegt D zwischen 5 und 40 %;
Wenn R strikt größer als 1 und kleiner oder gleich 5 ist, liegt D zwischen 4 und 30 %;
Wenn R strikt größer als 0,1 und kleiner oder gleich 1 ist, liegt D zwischen 3 und 20 %;
Wenn R strikt größer als 0,01 und kleiner oder gleich 0,1 ist, liegt D zwischen 1 und 6 %;

Wenn R kleiner oder gleich 0,01 ist, liegt D zwischen 1 und 3 %.

**12.** Verwendung des Polymers nach einem der Ansprüche 1 bis 11 in einem Bereich, der aus Öl- und Gasgewinnung, Wasseraufbereitung, Schlammbehandlung, der Papierherstellung, Bauwesen, Bergbau, Formulierung von Kosmetika, Formulierung von Waschmitteln, Textilherstellung und Landwirtschaft ausgewählt ist.

**13.** Verwendung des Polymers nach einem der Ansprüche 1 bis 12 als Flockungsmittel, Viskositätsreduktionsmittel, Verdickungsmittel, Absorptionsmittel, Reibungsreduktionsmittel oder Weichmacher oder Betonverflüssiger.

**Claims**

**1.** A polymer obtained at least from a quantity A of 2-acrylamido-2-methylpropane sulfonic acid, in acid and/or salified form, ***characterized* in that** quantity A of 2-acrylamido-2-methylpropane acid sulfonic acid contains 250 to 20,000 ppm by weight of 2-methyl-2-propenyl-sulfonic acid, in acid and/or salified form and between 300 and 10,000 ppm by weight of 2-methylidene-1,3-propylenedisulfonic acid.

**2.** The polymer according to Claim 1, ***characterized* in that** quantity A of 2-acrylamido-2-methylpropane sulfonic acid, in acid and/or salified form, contains 300 to 20,000 ppm of 2-methyl-2- propenyl-sulfonic, in acid and/or salified form.

**3.** The polymer according to claim 1, ***characterized* characterized in that** quantity A of 2-acrylamido-2-methylpropanesulfonic acid, in acid and/or salified form, contains 300 to 10,000 ppm of 2-methyl-2-propenylsulfonic acid, in acid and/or salified form.

**4.** The polymer according to one of Claims 1 to 3, ***characterized* in that** the polymer is further obtained from a quantity B of 2-acrylamido-2-methylpropane sulfonic acid, in acid and/or salified form, containing strictly less than 200 ppm by weight of 2-methyl-2-propenyl sulfonic acid, in acid and/or salified form.

**5.** The polymer according to one of Claims 1 to 4, ***characterized* in that** the polymer is a water-soluble polymer or a superabsorbent polymer.

**6.** The polymer according to one of Claims 1 to 5, ***characterized* in that** the polymer is water-soluble and has a weight-average molecular weight greater than 100,000 g/mol and less than or equal to 40 million g/mol, the molecular weight being determined by the intrinsic viscosity of the polymer.

**7.** The polymer according to one of Claims 1 to 6, ***characterized* in that** the polymer is a homopolymer of 2-acrylamido-2-methylpropane sulfonic acid, in acid and/or salified form, or a copolymer of 2-acrylamido-2-methylpropane sulfonic acid, in acid and/or salified form, further comprising at least one monomer chosen from the group comprising nonionic monomers, anionic monomers, cationic monomers, zwitterionic monomers, and mixtures thereof.

**8.** A polymer according to one of claims 1 to 7, ***characterized* in that** the polymer is obtained at least from 2-acrylamido-2-methylpropane sulfonic acid, in acid and/or salified form, the total quantity of which contains strictly less than 20,000 ppm by weight of 2-methyl-2-propenyl sulfonic acid, in acid and/or salified form.

**9.** The polymer according to one of Claims 1 to 8, ***characterized* in that** the polymer is water-soluble and **in that** the proportion D of quantity A relative to the total quantity of 2-acrylamido-2-methylpropane sulfonic acid, in acid and/or salified form, is determined from the ratio R defined according to the following equation (1):

$$R= \frac{10^{14}}{[IBSA]^2 * C^2 * Mw} \quad (1)$$

wherein,

[IBSA] is the concentration in ppm by weight in quantity A of 2-methyl-2-propenyl-1-sulfonic acid;
Mw is the weight-average molecular weight of the polymer expressed in g/mol;

C is a coefficient equal to 0.2 when Mw is less than or equal to 1 million, equal to 0.6 when Mw is strictly greater than 1 million and less than or equal to 10 million, and equal to 0.8 when Mw is strictly greater than 10 million;
when R is strictly greater than 100, D is between 1 and 100%;
when R is strictly greater than 50 and less than or equal to 100, D is between 1 and 90%;
when R is strictly greater than 10 and less than or equal to 50, D is between 1 and 80%;
when R is strictly greater than 5 and less than or equal to 10, D is between 1 and 60%;
when R is strictly greater than 1 and less than or equal to 5, D is between 1 and 50%;
when R is strictly greater than 0.1 and less than or equal to 1, D is between 1 and 30%;
when R is strictly greater than 0.01 and less than or equal to 0.1, D is between 1 and 10%;
when R is less than or equal to 0.01, D is between 1 and 5%.

**10.** The polymer according to Claim 9, ***characterized*** **in that**

when R is strictly greater than 100, D is between 25 and 95%;
when R is strictly greater than 50 and less than or equal to 100, D is between 25 and 85%;
when R is strictly greater than 10 and less than or equal to 50, D is between 25 and 75%;
when R is strictly greater than 5 and less than or equal to 10, D is between 2 and 50%;
when R is strictly greater than 1 and less than or equal to 5, D is between 2 and 40%;
when R is strictly greater than 0.1 and less than or equal to 1, D is between 2 and 25%;
when R is strictly greater than 0.01 and less than or equal to 0.1, D is between 1 and 8%;
when R is less than or equal to 0.01, D is between 1 and 4%.

**11.** The polymer according to Claim 9, ***characterized*** **in that**

when R is strictly greater than 100, D is between 50 and 90%;
when R is strictly greater than 50 and less than or equal to 100, D is between 50 and 80%;
when R is strictly greater than 10 and less than or equal to 50, D is between 50 and 70%;
when R is strictly greater than 5 and less than or equal to 10, D is between 5 and 40%;
when R is strictly greater than 1 and less than or equal to 5, D is between 4 and 30%;
when R is strictly greater than 0.1 and less than or equal to 1, D is between 3 and 20%;
when R is strictly greater than 0.01 and less than or equal to 0.1, D is between 1 and 6%;
when R is less than or equal to 0.01, D is between 1 and 3%.

**12.** Use of the polymer of one of claims 1 to 11 in a field selected from oil and gas recovery, water treatment, sludge treatment, papermaking, construction, mining, cosmetic formulation, detergent formulation, textile manufacturing, and agriculture.

**13.** Use of the polymer according to one of Claims 1 to 12 as a flocculant, viscosity-reducing agent, thickening agent, absorbing agent, friction-reducing agent or plasticizer or superplasticizer.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 20100274048 A
- JP 2011046612 A
- JP 2015504441 A
- JP 2010270169 A
- JP 2010270170 A
- JP 2010270168 A
- WO 2018172676 A